# EUROPEAN PATENT SPECIFICATION

(11) **EP 4 164 595 B1**
(45) Date of publication and mention of the grant of the patent: **20.08.2025**
(21) Application number: 21730990.5
(22) Date of filing: 28.05.2021
(51) Int. Cl.: A61K 9/00, A61K 9/16, A61P 5/16, A61P 25/30, A61P 29/00

(54) **ANATABINE POWDER COMPOSITIONS**
ANATABINPULVERZUSAMMENSETZUNGEN
COMPOSITIONS DE POUDRE D'ANATABINE

(30) Priority: 15.06.2020 EP 20180100
(43) Date of publication of application: 19.04.2023
(73) Proprietor: Philip Morris Products S.A., 2000 Neuchâtel (CH)
(72) Inventor: SPADARO, Fabiana, 2000 Neuchâtel (CH); ZUBER, Gérard, 2000 Neuchâtel (CH)
(74) Representative: Reddie & Grose LLP
(86) International application number: PCT/IB2021/054692
(87) International publication number: WO 2021/255560

(56) References cited:
- WO-A1-2018/002779
- WO-A2-2011/119722

## Description

The present disclosure relates to anatabine powder compositions with a sugar or adhesion reducing compound that may be suitable for inhalation and methods of forming the same. Sugar and an anatabine compound may be spray dried to form a dry anatabine powder composition for inhalation.

Dry powder inhalers (DPI) are known and are used to treat respiratory diseases by delivering a dry powder comprising a pharmaceutically active compound, in aerosol form through inhalation to airways of patients. In pharmaceutical dry powders, the active pharmaceutical ingredient (API) is usually agglomerated on the surface of larger carrier particles, such as lactose for example. DPI's operate through complex mechanisms to ensure such agglomerates disperse, break up or disaggregate before the API is inhaled into the lungs.

WO 2011/119722 A describes pharmaceutical compositions comprising an isolated form of anatabine, or a salt thereof, that can be used to treat disorders comprising an inflammatory component.

It would be desirable to provide an anatabine powder composition that is free-flowing and efficiently formed. It would be desirable to provide an anatabine powder composition that is sized to be delivered to the lungs or airway of a user via inhalation. It would be desirable to provide an anatabine powder composition that possesses a long shelf life.

The present invention is defined by the appended independent claims.

According to the present invention, a powder composition includes a plurality of particles comprising an anatabine compound and a sugar or adhesion reducing compound, and the plurality of particles have a particle size of about 20 micrometres or less, or about 10 micrometres or less, or from about 1 to about 4 micrometres, measured as mass medium aerodynamic diameter.

According to the present invention, a powder composition includes a plurality of particles comprising an anatabine compound and a sugar or adhesion reducing compound and the plurality of particles have a particle size of about 20 micrometres to about 200 micrometres, measured as mass medium aerodynamic diameter.

The plurality of particles may comprise an amorphous sugar.

The adhesion reducing compound may comprise an amino acid or peptide.

According to the present invention, a method of forming a powder composition comprises combining an anatabine compound and a sugar, in a liquid carrier, to form a liquid mixture, and spray drying the liquid mixture to form a plurality of particles, or spray drying the liquid mixture to form a plurality of particles having a first size, and then micronizing the plurality of particles having a first size to a reduced size.

Spray drying the liquid mixture to form a plurality of particles may comprise spray drying the liquid mixture to form a plurality of particles having a size of from about 20 micrometres or less, or about 10 micrometres or less, or from about 1 to about 4 micrometres, measured as mass medium aerodynamic diameter.

Spray drying the liquid mixture to form a plurality of particles may comprise spray drying the liquid mixture to form a plurality of particles having a size of from about 20 micrometres to about 200 micrometres, measured as mass medium aerodynamic diameter.

Micronizing the plurality of particles having a first size to a reduced size may comprise micronizing the plurality of particles having a first size to a reduced size in a range from about 20 micrometres or less, or from about 10 micrometres or less, or from about 1 to about 4 micrometres, measured as mass medium aerodynamic diameter.

Micronizing the plurality of particles having a first size to a reduced size may comprise micronizing the plurality of particles having a first size to a reduced size in a range from about 20 micrometres to about 200 micrometres, or from about 50 to about 150 micrometres, measured as mass medium aerodynamic diameter.

Advantageously, the anatabine powder composition may be a free-flowing powder. Advantageously, the anatabine powder composition may be free-flowing and may be sized to be delivered to the lungs or airway of a user via inhalation. The anatabine powder composition may be stable and possesses a long shelf life. The methods result in dry powder anatabine particles that are free-flowing and may be suitable for inhalation, for example. The method may enable high throughput manufacturing of the anatabine powder composition without compromising the physical and chemical properties of the anatabine powder composition.

The spray drying methods form composite anatabine particles each containing anatabine and a matrix compound such as sugar. The composite anatabine particles form the powder compositions described herein. The anatabine may be dispersed within the sugar matrix. Preferably the anatabine is a solid salt at 25 °C.

The spray drying methods form composite anatabine particles each containing anatabine and a matrix compound such as sugar and an adhesion reducing compound coating, preferably an amino acid. The anatabine may be dispersed within the sugar matrix and an amino acid, such as leucine, may coat or encapsulate each composite anatabine particle forming a free-flowing composite anatabine particle composition. Preferably the anatabine is a solid salt at 25 °C.

Anatabine is an alkaloid present in tobacco and, in lower concentrations, in a variety of foods, including green tomatoes, green potatoes, ripe red peppers, tomatillos, and sundried tomatoes. It is a main active component of the marketed dietary supplement anatabloc providing anti-inflammatory support, as disclosed in US 9,387,201 and WO 2013/032558. The preparation of isolated forms of anatabine is described in WO 2011/119722, for example.

Anatabine is also known as 3-(1,2,3,6-tetrahydropyridin-2-yl)pyridine. Enantioselective syntheses of S- and R-enantiomers of anatabine are described, for example, in Ayers, J. T.; Xu, R.; Dwoskin, L. P.; Crooks, P. A. A general procedure for the enantioselective synthesis of the minor Tobacco alkaloids nornicotine, anabasine, and anatabine. The AAPS Journal 2005; 7(3) Article 75.

The term "anatabine" as used here may refer to (1) a racemic mixture of anatabine (R,S); (2) a purified form of S-(-)-anatabine; or (3) a purified form of R-(+)-anatabine. A preferred anatabine compound is anatabine salt such as anatabine glutarate or 3-(1,2,3,6-tetrahydropyridin-2-yl)pyridine glutarate. Preferably, the 3-(1,2,3,6-tetrahydropyridin-2-yl)pyridine glutarate has a 1:1 molar ratio of 3-(1,2,3,6-tetrahydropyridin-2-yl)pyridine to glutarate.

Pharmaceutically acceptable salts of anatabine are described in US Pat. 8,207,346 and US Pat. 8,557,999. In particular, Example 6 of US Pat. 8,207,346 and Example 6 of US Pat. 8,557,999 describe the preparation of anatabine tartrate and anatabine citrate by addition of tartaric acid or citric acid to a solution of anatabine in acetone.

It is to be understood that any reference to "3-(1,2,3,6-tetrahydropyridin-2-yl)pyridine glutarate" or "anatabine glutarate" herein is to be understood as also referring to any pharmaceutically acceptable solvate thereof.

The 3-(1,2,3,6-tetrahydropyridin-2-yl)pyridine glutarate has a chemical structure represented by the following formula (I):

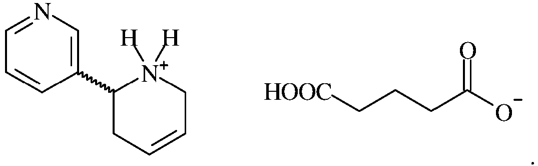

In a preferred embodiment, the 3-[1,2,3,6-tetrahydropyridin-2-yl]pyridine glutarate may thus have the following formula (la):

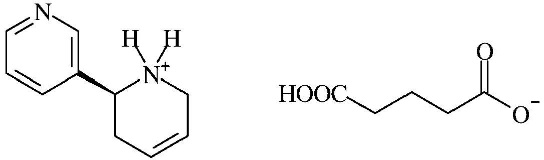

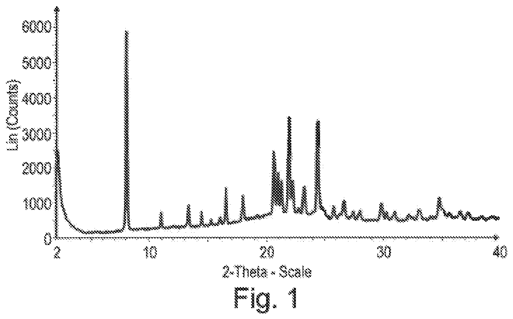

Preferably the anatabine glutarate is a specific polymorph (herein also referred to as polymorphic form) of the 3-(1,2,3,6-tetrahydropyridin-2-yl)pyridine glutarate and in particular of the crystal of the 3-(1,2,3,6-tetrahydropyridin-2-yl)pyridine glutarate. The polymorph preferably has an X-ray powder diffraction pattern (CuKα) substantially as shown in FIG. 1. The polymorph preferably has an X-ray powder diffraction pattern (CuKα) comprising one or more peaks selected from 8.0 ± 0.2 °2θ, 11.0 ± 0.2 °2θ, 13.3 ± 0.2 °2θ, 16.5 ± 0.2 °2θ, 18.0 ± 0.2 °2θ, 20.7 ± 0.2 °2θ, 21.0 ± 0.2 °2θ, 21.4 ± 0.2 °2θ, 22.0 ± 0.2 °2θ, 22.3 ± 0.2 °2θ, 23.3 ± 0.2 °2θ and 24.5 ± 0.2 °2θ. More preferably, the polymorph preferably has an X-ray powder diffraction pattern (CuKα) comprising one or more peaks selected from 8.0 ± 0.2 °2θ, 13.3 ± 0.2 °2θ, 16.5 ± 0.2 °2θ, 21.4 ± 0.2 °2θ, 22.0 ± 0.2 °2θ and 24.5 ± 0.2 °2θ.

Still more preferably, the polymorph preferably has an X-ray powder diffraction pattern (CuKα) comprising one or more peaks selected from 8.0 ± 0.1 °2θ, 11.0 ± 0.1 °2θ, 13.3 ± 0.1 °2θ, 16.5 ± 0.1 °2θ, 18.0 ± 0.1 °2θ, 20.7± 0.1 °2θ, 21.0 ± 0.1 °2θ, 21.4 ± 0.1 °2θ, 22.0 ± 0.1 °2θ, 22.3 ± 0.1 °2θ, 23.3 ± 0.1 °2θ and 24.5 ± 0.1 °2θ. Even more preferably, the polymorph preferably has an X-ray powder diffraction pattern (CuKα) comprising one or more peaks selected from 8.0 ± 0.1 °2θ, 13.3 ± 0.1 °2θ, 16.5 ± 0.1 °2θ, 21.4 ± 0.1 °2θ, 22.0 ± 0.1 °2θ and 24.5 ± 0.1 °2θ.

Even more specifically, the polymorph preferably has an X-ray powder diffraction pattern (CuKα) comprising one or more peaks selected from 7.960 ± 0.2 °2θ, 10.907 ± 0.2 °2θ, 13.291 ± 0.2 °2θ, 14.413 ± 0.2 °2θ, 15.239 ± 0.2 °2θ, 16.479 ± 0.2 °2θ, 17.933 ± 0.2 °2θ, 20.610 ± 0.2 °2θ, 20.977 ± 0.2 °2θ, 21.318 ± 0.2 °2θ, 21.927 ± 0.2 °2θ, 22.203 ± 0.2 °2θ, 22.792 ± 0.2 °2θ, 23.246 ± 0.2 °2θ, 24.426 ± 0.2 °2θ and 24.769 ± 0.2 °2θ. Still more specifically, the polymorph preferably has an X-ray powder diffraction pattern (CuKα) comprising one or more peaks selected from 7.960 ± 0.1 °2θ, 10.907 ± 0.1 °2θ, 13.291 ± 0.1 °2θ, 14.413 ± 0.1 °2θ, 15.239 ± 0.1 °2θ, 16.479 ± 0.1 °2θ, 17.933 ± 0.1 °2θ, 20.610 ± 0.1 °2θ, 20.977 ± 0.1 °2θ, 21.318 ± 0.1 °2θ, 21.927 ± 0.1 °2θ, 22.203 ± 0.1 °2θ, 22.792 ± 0.1 °2θ, 23.246 ± 0.1 °2θ, 24.426 ± 0.1 °2θ and 24.769 ± 0.1 °2θ.The above form of anatabine glutarate may be prepared using a method comprising the steps of:
a) preparing a solution comprising 3-[1,2,3,6-tetrahydropyridin-2-yl]pyridine, glutaric acid and a solvent,
b) allowing the formation of a salt of 3-[1,2,3,6-tetrahydropyridin-2-yl]pyridine with the glutaric acid, and
c) recovering the 3-[1,2,3,6-tetrahydropyridin-2-yl]pyridine glutaric acid salt.

The solvent used in the preparation of the solution of 3-[1,2,3,6-tetrahydropyridin-2-yl]pyridine, glutaric acid and a solvent preferably comprises 2-methyltetrahydrofuran, acetonitrile and/or ethyl acetate. More preferably, the solvent comprises 2-methyltetrahydrofuran.

The method may furthermore comprise a step of d) recrystallizing the 3-[1,2,3,6-tetrahydropyridin-2-yl]pyridine glutaric acid salt. Suitable solvents for this recrystallization include acetonitrile.

In step a), the anatabine glutarate can be prepared by combining anatabine free base, a solvent, and glutaric acid to create a reaction mixture. Anatabine glutarate typically forms in such a reaction mixture through contact of anatabine free base with glutaric acid. Preferably, anatabine free base as a 1 to 5 mass-% solution in acetonitrile is combined with glutaric acid.

Preferably a solution or suspension of anatabine free base, a solvent and glutaric acid is combined to form a reaction mixture, followed by precipitation and recovery of the anatabine glutarate salt from the mixture. Glutaric acid may be added either as a solid or as a solution or a suspension in a solvent.

The solvent is preferably selected from the group consisting of alkanols containing 1 to 8 carbon atoms, aliphatic esters containing 3 to 8 carbon atoms, aliphatic linear or cyclic ethers containing 3 to 8 carbon atoms, aliphatic ketones containing 3 to 8 carbon atoms, C₆₋₁₂ aromatic hydrocarbons (such as benzene and napthalene), acetonitrile, water, and any mixtures thereof. Preferably, the solvent is selected from aliphatic esters containing 3 to 8 carbon atoms, aliphatic cyclic ethers containing 3 to 8 carbon atoms, acetonitrile and a mixture thereof. More preferably, the solvent is selected from ethyl acetate, acetonitrile, 2-methyltetrahydrofuran, and any mixtures thereof. Even more preferably, the solvent contains acetonitrile. Still more preferably, the solvent is acetonitrile.

The anatabine free base, glutaric acid, and the at least one solvent are preferably combined to form the reaction mixture at about room temperature (i.e. a range of preferably 15°C to 25°C). The concentration of glutaric acid present in such reaction mixture is preferably a concentration close to the point of saturation (e.g. at least 80%, preferably 90%, more preferably 95% of the maximum achievable concentration). Anatabine glutarate typically precipitates out of the mixture. The precipitation may occur on its own or be induced, e.g., by the introduction of seed crystals. The reaction mixture may be stirred before, during, or after precipitation.

The reaction mixture may be heated and then cooled to facilitate precipitation of anatabine glutarate. Heating may be carried out up to any temperature (e.g. about 50°C to about 80°C) in the range of from room temperature to the boiling temperature of the solvent. Thereafter, cooling is generally conducted down to less than 40°C, preferably about 30°C to about 20°C, more preferably room temperature (i.e. a range of preferably 15°C to 25°C), to facilitate precipitation.

The resulting precipitate may be recovered by various techniques, such as filtration. The precipitate may be dried under ambient or reduced pressure and/or elevated temperature.

Anatabine glutarate, and particularly the polymorphic form described above, has advantageous properties such as high crystallinity, morphology, thermal and mechanical stability to polymorphic conversion and/or to dehydration, storage stability, low content of residual solvent, a lower degree of hygroscopicity, flowability, and advantageous processing and handling characteristics. Furthermore, anatabine glutarate recrystallizes as a crystalline salt even after having been exposed to moisture, when the moisture is removed by suitable measures, such as drying under vacuum.

Anatabine (for example, anatabine glutarate) can be administered to an individual to treat disorders comprising an inflammatory component, including chronic, low-level inflammation. Anatabine can be administered to an individual to reduce a symptom or a disorder comprising an NFKB-mediated inflammatory component and/or to reduce the risk of developing such a disorder. The NFKB-mediated inflammatory component may be associated with chronic inflammation which occurs, for example, in thyroiditis, cancer, arthritis, Alzheimer's disease, and multiple sclerosis. The inhalable powder comprising anatabine may have a monoamine oxidase (MAO) inhibitory effect. Additionally, or alternatively, the inhalable powder comprising anatabine may have a STAT3 phosphorylation inhibition effect.

In some embodiments, the anatabine is formulated as a salt. Any pharmaceutically acceptable salt may be used. Preferably, the anatabine salt is solid at room temperature (for example, solid at 25 °C). Suitable salts include, for example, a salt of aspartic acid ("aspartate"), gentisic acid ("gentisate"), benzoic acid ("benzoate"), fumaric acid ("fumarate"), hydrochloric acid ("hydrate"), alfa-resorcylic acid ("alfa-resorcylate"), beta-resorcylic acid ("beta-resorcylate"), oxalic acid ("oxalate"), p-anisic acid ("anisate"), or glutaric acid ("glutarate"). Preferably the salt comprises glutarate, such as anatabine glutarate. Preferably, the anatabine salt is anatabine glutarate. Preferably, the anatabine glutarate is the polymorphic form described above.

Anatabine or an anatabine compound may be present in the powder composition in an amount from 0.1 to 30% anatabine compound by weight, or from 0.5 to 20% anatabine compound by weight. Anatabine or an anatabine compound may be present in the powder composition in an amount from 1 to 10% anatabine compound by weight. Anatabine or an anatabine compound may be present in the powder composition in an amount from 10 to 20% anatabine compound by weight. Anatabine or an anatabine compound may be present in the powder composition in an amount from 1 to 5% anatabine compound by weight. Anatabine or an anatabine compound may be present in the powder composition in an amount from 5 to 15% anatabine compound by weight.

The plurality of particles may include sugar. Sugar may form a matrix for dispersing anatabine throughout the particle forming the powder composition. Sugar and anatabine may form a composite particle.

The sugar may preferably be an amorphous sugar. The plurality of particles may include a monosaccharide, disaccharide, polysaccharide, or mixtures thereof. The plurality of particles may include lactose, sucrose, raffinose, trehalose, fructose, dextrose, glucose, maltose, or combinations thereof. The plurality of particles may preferably include trehalose.

The plurality of particles (or each particle) may include from 50 to 99% sugar by weight. The plurality of particles (or each particle) may include from 70 to 90% sugar by weight. The plurality of particles (or each particle) may include from 70 to 80% sugar by weight. The plurality of particles (or each particle) may include from 80 to 90% sugar by weight. The plurality of particles (or each particle) may include from 80 to 85% sugar by weight.

The powder composition may comprise an adhesion reducing compound to reduce agglomeration of particles and ensure or promote a free-flowing powder composition. The adhesion reducing compound may reduce the adhesion or cohesion experienced by the particles of the powder composition. The additive material may interfere with the weak bonding forces between the small particles, helping to keep the particles separated and reducing the adhesion of such particles to one another, to other particles in the formulation and to internal surfaces of an associated inhaler device.

The adhesion reducing compound may include a phospholipid or a derivative thereof such as lecithin.

The adhesion reducing compound may include a metal stearate, or a derivative thereof, for example, sodium stearyl fumarate or sodium stearyl lactylate. Useful metal stearates include, for example, zinc stearate, magnesium stearate, calcium stearate, sodium stearate or lithium stearate. Preferably, the adhesion reducing compound includes magnesium stearate.

The adhesion reducing compound may include or consist of one or more surface active materials, in particular materials that are surface active in the solid state, which may be water soluble or water dispersible, for example lecithin, in particular soya lecithin, or substantially water insoluble, for example solid state fatty acids such as oleic acid, lauric acid, palmitic acid, stearic acid, erucic acid, behenic acid, or derivatives (such as esters and salts) thereof such as glyceryl behenate. Specific examples of such materials are: phosphatidylcholines, phosphatidylethanolamines, phosphatidylglycerols and other examples of natural and synthetic lung surfactants; lauric acid and its salts, for example, sodium lauryl sulphate, magnesium lauryl sulphate; triglycerides such as Dynsan 118 and Cutina HR; and sugar esters in general. Alternatively, the additive may be cholesterol.

Other possible adhesion reducing compounds include sodium benzoate, hydrogenated oils which are solid at room temperature, talc, titanium dioxide, aluminium dioxide, silicon dioxide and starch.

The adhesion reducing compound may include an amino acid or peptide (containing three amino acids, for example). The amino acid may comprise histidine, alanine, isoleucine, arginine, leucine, asparagine, lysine, aspartic acid, methionine, cysteine, phenylalanine, glutamic acid, threonine, glutamine, tryptophan, glycine, valine, pyrrolysine, proline, selenocysteine, serine, tyrosine, or a combination thereof. Preferably the amino acid comprises leucine, alanine, valine, isoleucine, methionine, phenylalanine, tyrosine, tryptophan, or a combination thereof. Preferably the amino acid comprises leucine, such as L-leucine. Preferably the peptide, if present, comprises trileucine.

The anatabine containing particles may be coated by an adhesion reducing compound, such as an amino acid or peptide or metal stearate. The anatabine containing particles may include an adhesion reducing compound, such as an amino acid or peptide or metal stearate dispersed throughout the particles forming the powder composition.

Amino acid or an adhesion reducing compound, such as an amino acid or peptide or metal stearate acid may be added to the anatabine containing particles after spray drying. For example, the particles formed by spray drying may be mixed with the adhesion reducing compound, and the mixture may be co-milled (for example, micronized). The co-milling may enable the amino adhesion reducing compound to coat the anatabine containing particles. Co-milling may further achieve a desired final particle size (for example, reduced from a particle size of about 50 µm to about 2 µm).

The anatabine containing particles may comprise 5 wt-% or more or 10 wt-% or more of adhesion reducing compound, and 30 wt-% or less or 25 wt-% or less of adhesion reducing compound by weight of the anatabine containing particles. The plurality of particles may include from 0.5 to 20% adhesion reducing compound by weight. The plurality of particles may include from 1 to 15% adhesion reducing compound by weight. The plurality of particles may include from 1 to 10% adhesion reducing compound by weight.

The anatabine containing particles may comprise 5 wt-% or more or 10 wt-% or more of an amino acid or peptide, and 30 wt-% or less or 25 wt-% or less of amino acid or peptide by weight of the anatabine containing particles. The plurality of particles may include from 0.5 to 20% amino acid or peptide by weight. The plurality of particles may include from 1 to 15% amino acid by weight. The plurality of particles may include from 1 to 10% amino acid by weight.

Providing an adhesion reducing compound such as an amino acid, preferably L-leucine with the anatabine containing particles may reduce adhesion forces of the anatabine containing particles and may reduce attraction between the particles and thus further reduce agglomeration of the particles. Similarly, powder systems that include other particles, such as a plurality of second particles that may comprise flavor, adhesion forces to the other particles may also be reduced thus agglomeration of the anatabine containing particles with other particles is also reduced. The other particles, such as plurality of second particles that may comprise flavor, if present, may include adhesion reducing compound such as an amino acid, preferably L-leucine to further reduce adhesion forces of the anatabine containing particles and the other particles. The powder system described herein thus may be a free-flowing material and possess a stable relative particle size of each powder component even when the anatabine containing particles and the other particles are combined.

The anatabine containing particles may include 0.5 to 20% anatabine glutarate, 70 to 90% sugar, and 0 to 20% adhesion reducing compound, all by weight. The anatabine containing particles may preferably include 0.5 to 20% anatabine glutarate, 70 to 90% sugar, and 1 to 20% adhesion reducing compound, all by weight. The anatabine containing particles may preferably include 1 to 10% anatabine glutarate, 70 to 90% sugar, and 1 to 10% adhesion reducing compound, all by weight.

The anatabine containing particles may include 0.5 to 20% anatabine glutarate, 70 to 90% sugar, and 0 to 20% leucine, all by weight. The anatabine containing particles may preferably include 0.5 to 20% anatabine glutarate, 70 to 90% sugar, and 1 to 20% leucine, all by weight. The anatabine containing particles may preferably include 1 to 10% anatabine glutarate, 70 to 90% sugar, and 1 to 10% leucine, all by weight.

The anatabine containing particles may have a particle size of 20 micrometres or less, 10 micrometres or less, or 5 micrometres or less, or 0.1 micrometres or greater, 0.2 micrometres or greater, or 0.5 micrometres or greater, or ranging from 0.5 micrometres to 10 micrometres or from 0.75 micrometres to 5 micrometres. The desired particle size range may be achieved by spray drying, milling, sieving, or a combination thereof. This size range may be useful for respiratory deposition, such as deep lung deposition of the anatabine containing particles.

The anatabine containing particles may have a particle size of 20 micrometres or less, 10 micrometres or less, or 5 micrometres or less, or 1 micrometres or greater, or 2 micrometres or greater, or 5 micrometres or greater, or ranging from 5 micrometres to 10 micrometres or from 7.5 micrometres to 10 micrometres. The desired particle size range may be achieved by spray drying, milling, sieving, or a combination thereof. This size range may be useful for respiratory deposition, such as bronchi deposition of the anatabine containing particles.

The anatabine containing particles may have a particle size of 20 micrometres or greater, or from 20 to 200 micrometres, or from 50 to 200 micrometres, or from 50 to 150 micrometres. The desired particle size range may be achieved by spray drying, milling, sieving, or a combination thereof. This size range may be useful for upper respiratory deposition, such as sinus or buccal deposition of the anatabine containing particles.

The anatabine containing particles may have a specific particle size distribution. In illustrative examples, about 90%, or about 95%, or about 98% of the anatabine containing particles of the composition have a size of about 5 micrometres or less, or about 4.5 micrometres or less, or about 4.2 micrometres or less, and about 50% of the particles have a size of about 2.5 micrometres or less, or about 2.1 micrometres or less. In many of these examples, about 10% of the anatabine containing particles have a size of about 820 nanometers or less. The anatabine containing particles of the composition may have a mass median aerodynamic diameter in a range from about 1 to about 4 micrometres. Substantially all of the anatabine containing particles forming the composition may have a particle size in a range from about 500 nanometers to about 5 micrometres. The percentages relating to particle size distribution described herein are based on particles by volume (% by volume).

The anatabine containing particles may be further mixed or combined with a second plurality of particles. In some embodiments, the second plurality of particles have a different particle size than the anatabine containing particles and form a second population of particles that are larger than the anatabine containing particles. The second plurality of particles may be useful in controlling or aiding in delivery of the anatabine particles. The second plurality of particles may include a flavor compound. The second plurality of particles may have any useful size distribution for inhalation delivery selectively into the mouth or buccal cavity of a user. For example, the second plurality of particles may have a particle size of about 20 micrometres or greater, or about 50 micrometres or greater, 200 micrometres or smaller, 150 micrometres or smaller, or in a range from 50 micrometres to 200 micrometres, or from 50 micrometres to 150 micrometres. Alternatively, where the anatabine particles are suitable sized for deposition in the mouth or buccal cavity they may comprise flavor compounds.

The powder system may comprise a weight ratio of the first plurality of anatabine containing particles to second plurality of particles of about 1:1 to about 10:1, or about 2:1 to about 8:1, or about 2:1 to about 6:1, or about 3:1 to about 5:1, preferably about 4:1. By selecting the weight ratio of the first plurality of anatabine containing particles to second plurality of particles, it is possible to improve the delivery of the content of a container (such as a capsule) containing the powder system over a series of inhalations containing a similar amount of powder.

The powder system may comprise a weight ratio of the first plurality of anatabine containing particles to second plurality of flavor particles of about 1:1 to about 10:1, or about 2:1 to about 8:1, or about 2:1 to about 6:1, or about 3:1 to about 5:1, preferably about 4:1. By selecting the weight ratio of the first plurality of anatabine containing particles to second plurality of flavor particles, it is possible to improve the delivery of the content of a container (such as a capsule) containing the powder system over a series of inhalations containing a similar amount of powder.

The powder system is preferably free-flowing. The first plurality of anatabine containing particles is preferably free-flowing. The second plurality of particles is preferably free-flowing. The powder system contained within a container or capsule is preferably free-flowing. The first plurality of anatabine containing particles contained within the container or capsule is preferably free-flowing. The second plurality of particles contained within the container or capsule is preferably free-flowing. The powder system may have a stable size distribution. The powder system preferably does not agglomerate.

The second plurality of particles may be free of anatabine. The second plurality of particles may include a flavor. Suitable flavors include, but are not limited to, any natural or synthetic flavor, such as tobacco, smoke, menthol, mint (such as peppermint and spearmint), chocolate, licorice, citrus and other fruit flavors, gamma octalactone, vanillin, ethyl vanillin, breath freshener flavors, spice flavors such as cinnamon, methyl salicylate, linalool, bergamot oil, geranium oil, lemon oil, and ginger oil, and the like. Other suitable flavors may include flavor compounds selected from the group consisting of an acid, an alcohol, an ester, an aldehyde, a ketone, a pyrazine, combinations or blends thereof and the like. Suitable flavor compounds may be selected, for example, from the group consisting of phenylacetic acid, solanone, megastigmatrienone, 2-heptanone, benzylalcohol, cis-3-hexenyl acetate, valeric acid, valeric aldehyde, ester, terpene, sesquiterpene, nootkatone, maltol, damascenone, pyrazine, lactone, anethole, iso-s valeric acid, combinations thereof, and the like.

Flavorants or flavors may be provided as a solid flavor (at room temperature of about 22 degrees centigrade and one atmosphere pressure) and may include flavor formulations, flavor-containing materials and flavor precursors. The flavorant may include one or more natural flavorants, one or more synthetic flavorants, or a combination of natural and synthetic flavorants.

The flavor compound/component may be derived from natural flavoring substances, nature-identical flavoring substances, or artificial flavoring substances. Non-limiting examples of flavor components, or flavors, include banana, cherry, cinnamon, fruit, grape, orange, pear, pineapple, vanilla, wintergreen, strawberry, and mint. In one embodiment, the flavor is menthol. In another embodiment, the flavor is mint. As one skilled in the art would understand, mint refers generally, but without being limited, to any and all flavors associated with the genus of plants in the family Lamiaceae. In one embodiment, mint is a natural extract. In another embodiment, mint is a commercially available formulation, such as for example Coolmint Trusil Flavoring Powder, supplied by International Flavors & Fragrances. In one embodiment, mint is one substance. In another embodiment, mint is a mixture of substances. In one embodiment, mint comprises menthol. In another embodiment, mint comprises trans-menthone. In another embodiment, mint comprises pinene. In another embodiment, mint comprises isomenthone. In another embodiment, mint comprises limonene. In another embodiment, mint comprises eucalyptol. In another embodiment, mint comprises pin-2(3)-ene. In another embodiment, mint comprises menthyl acetate. In another embodiment, mint comprises cineole. In another embodiment, mint comprises 4,5,6,7-tetrahydro3,6-dimethylbenzofuran. In another embodiment, mint comprises pin-2(10)-ene. In another embodiment, mint comprises dipentene. In another embodiment, mint comprises d-limonene. In another embodiment, mint comprises (R)-p-mentha-I ,8-diene. Preferably the second plurality of particles include menthol or mint.

The second plurality of particles may include a compound to reduce adhesion forces or surface energy and any resulting agglomeration. The second plurality of particles may be surface modified with an adhesion reducing compound to form coated particles. The adhesion reducing compound may include an amino acid or peptide, as described above or magnesium stearate, or combinations thereof.

The amino acid to reduce adhesion forces or surface energy and any resulting agglomeration in the second plurality of particles may comprise histidine, alanine, isoleucine, arginine, leucine, asparagine, lysine, aspartic acid, methionine, cysteine, phenylalanine, glutamic acid, threonine, glutamine, tryptophan, glycine, valine, pyrrolysine, proline, selenocysteine, serine, tyrosine, or a combination thereof. Preferably the amino acid comprises leucine, alanine, valine, isoleucine, methionine, phenylalanine, tyrosine, tryptophan, or a combination thereof. Preferably the amino acid comprises leucine, such as L-leucine. Preferably the peptide comprises trileucine. The amino acid or peptide may preferably coat the particle forming the second plurality of particles. The amino acid or peptide may preferably coat the flavor particle.

One preferred adhesion reducing compound may be magnesium stearate. Providing an adhesion reducing compound such as magnesium stearate with the second plurality of particles, especially coating the second plurality of particles, may reduce adhesion forces of the second plurality of particles that may comprises flavor and may reduce attraction between particles and thus reduce agglomeration of particles. Agglomeration of second plurality of particles with the anatabine containing particles may also be reduced. The powder system may possess a stable relative particle size of the anatabine containing particles and the second plurality of particles even when the anatabine containing particles and the second plurality of particles are combined. The powder system preferably may be free-flowing.

The anatabine containing particles and second plurality of particles may be combined in any useful relative amount. Where the second plurality of particles comprise a flavor the may be combined in any useful relative amount so that the flavor particles are detected by the user when consumed with the anatabine containing particles. Preferably, the anatabine containing particles and second plurality of particles form at least about 90 wt-% or at least about 95 wt-% or at least about 99 wt-% or 100 wt-% of the total weight of the powder system.

The powder system can further comprise an excipient that is any pharmaceutically acceptable material, composition or carrier, such as a liquid or solid filler, stabilizer, dispersing agent, suspending agent, diluent, thickening agent, solvent or encapsulating material, involved in carrying or transporting a compound useful within the invention within or to the subject such that it may perform its intended function. In one embodiment, the formulation further comprises a stabilizing agent. Each material must be "acceptable" in the sense of being compatible with the other ingredients of the formulation, including anatabine, and not injurious to the subject. Some materials that may be useful in the formulation of the present invention include pharmaceutically acceptable carriers, for example sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, com oil and soybean oil; glycols, such as propylene glycol; polyols, such as glycerin, and polyethylene glycol; esters, such as ethyl oleate and ethyl laurate; agar; buffering agents, such as magnesium hydroxide and aluminum hydroxide; surface active agents; amino acids, such as leucine, L-leucine, D-leucine, DL-leucine, isoleucine, lysine, valine, arginine, aspartic acid, threonine, methionine, phenylalanine; alginic acid; derivatives of amino acids, such as derivative of an amino acid, for example aspartame or acesulfame K; pyrogen-free water; isotonic saline; Ringer's solution; ethyl alcohol; phosphate buffer solutions; and other nontoxic compatible substances employed in pharmaceutical formulations. Other pharmaceutically acceptable materials that can be useful in the formulation include any and all coatings, antibacterial and antifungal agents, and absorption delaying agents, and the like that are compatible with the activity of anatabine or any other compound useful within the invention, and are physiologically acceptable to the subject.

The powder composition may have pH (in solution) in a range recommended for human consumption. In some embodiments, the inhalable powder has a pH of 6 or less, 7 or less, or 8 or less, or between 4 and 8, or between 4 and 6, when dissolved in water. The pH of the powder composition may be tested by making up a 1.0 mg/mL solution of the powder in water and measuring the pH of the solution. The powder composition may be formulated without the use of an additional buffer. Additional buffering agents may be considered to be compounds capable of buffering (for example, salts, acids, bases, and combinations thereof) other than the acid used to form the salt with the active agent, or the amino acid included in the active-agent containing particles. The powder composition may be free of surfactants.

The powder composition may be provided in a suitable dosage form. For example, the powder composition may be provided in a capsule. The dosage form (for example, capsule) may be configured for use in a suitable inhaler or delivery device. For example, the capsule may be utilized in an inhaler device having a capsule cavity. Air flow management through a capsule cavity of the inhaler device may cause a capsule contained therein to rotate during inhalation and consumption. The capsule may contain the anatabine particles and optionally a plurality of second particles that may comprises flavor (also referred to as "flavor particles"). Rotation of a pierced capsule may suspend and aerosolize the powder composition released from the pierced capsule into the inhalation air moving through the inhaler device. The plurality of second particles, that may comprise flavor, may be larger than the anatabine containing particles and may assist in transporting the anatabine particles into the lungs of the user while the plurality of second particles, that may comprise flavor, preferentially remain in the mouth or buccal cavity of the user. The anatabine containing particles and optional plurality of second particles, that may comprises flavor, may be delivered with the inhaler device at inhalation or air flow rates that are within conventional smoking regime inhalation or air flow rates.

The capsule containing the powder composition or powder system may be formed of an airtight material that may be pierced or punctured by a piercing element that may be separate or combined with the inhaler. The capsule may be formed of a metallic or polymeric material that serves to keep contaminants out of the capsule but may be pierced or punctured by a piercing element prior to consumption of the anatabine particles within the capsule. The capsule may be formed of a polymer material. The polymer material may be hydroxypropylmethylcellulose (HPMC). The capsule may be a size 0 to size 5 capsule, or a size 2 capsule, or a size 3 capsule, or a size 4 capsule. The capsule may contain from 20 mg to 80 mg of inhalable powder, or from 40 mg to 60 mg of inhalable powder, or 50 mg of inhalable powder.

The term "particle size" is used here to refer to the mass median aerodynamic diameter (MMAD) of the particle or set of particles, unless otherwise stated. Such values are based on the distribution of the aerodynamic particle diameters defined as the diameter of a sphere with a density of 1 gm/cm³ that has the same aerodynamic behavior as the particle which is being characterized.

In particular, for a powder system reference is commonly made to the mass median aerodynamic diameter (MMAD), one of the metrics most widely adopted as a single number descriptor of aerodynamic particle-size distribution. The MMAD is a statistically derived figure for a particle sample: by way of example, an MMAD of 5 micrometres means that 50 percent of the total sample mass will be present in particles having aerodynamic diameters of less than 5 micrometres, and that the remaining 50 percent of the total sample mass will be present in particles having an aerodynamic diameter greater than 5 micrometres. In the context of the present invention, when describing a powder system, the term "particle size" preferably refers to the MMAD of the powder system.

The MMAD of a powder system is measured with a cascade impactor. Cascade impactors are instruments which have been extensively used for sampling and separating airborne particles for determining the aerodynamic size classification of aerosol particles. In practice, cascade impactors separate an incoming sample into discrete fractions on the basis of particle inertia, which is a function of particle size, density and velocity. A cascade impactor typically comprises a series of stages, each of which comprises a plate with a specific nozzle arrangement and a collection surface. As nozzle size and total nozzle area both decrease with increasing stage number, the velocity of the sample-laden air increases as it proceeds through the instrument. At each stage, particles with sufficient inertia break free from the prevailing air stream to impact on the collection surface. Therefore, at any given flow rate, each stage is associated with a cut-off diameter, a figure that defines the size of particles collected. With increasing stage number, velocity increases and so stage cut-off diameter decreases. Thus, the cut-off diameter associated with a given stage is a function of the air-flow rate used for testing. To reflect in-use performance, nebulisers are routinely tested at 15 L/min and dry powder inhalers may be tested at flow rates up to 100 L/min.

Preferably, in the context of the present invention, the MMAD of a powder system is measured with a Next Generation Impactor (NGI) 170 (available from Copley Scientific AG). The NGI is a high performance, precision, particle classifying cascade impactor having seven stages plus a Micro-Orifice Collector (MOC). Characteristics and operation principle of a NGI are described, for example, in Marple et al., Journal of Aerosol Medicine - Volume 16, Number 3 (2003). More preferably, measurements are carried out at 20 ±3 degrees Celsius and relative humidity of 35 ± 5 percent.

A dry powder formulation typically contains less than or equal to about 15 percent by weight moisture, preferably less than or equal to about 10 percent moisture, even more preferably less than or equal to about 6 percent by weight moisture. Most preferably a dry powder formulation contains less than or equal to about 5 percent by weight moisture or even less than or equal to about 3 percent by weight moisture or even less than or equal to about 1 percent by weight moisture.

All values reported as a percentage is presumed to be weight percent based on the total weight.

All scientific and technical terms used herein have meanings commonly used in the art unless otherwise specified. The definitions provided herein are to facilitate understanding of certain terms used frequently herein.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open-ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure, including the claims.

The term "substantially" as used here has the same meaning as "significantly," and can be understood to modify the relevant term by at least about 90 %, at least about 95 %, or at least about 98 %. The term "not substantially" as used here has the same meaning as "not significantly," and can be understood to have the inverse meaning of "substantially," i.e., modifying the relevant term by not more than 10 %, not more than 5 %, or not more than 2 %.

The present invention will now be further described with reference to the figure in which:
FIG. 1 is an X-ray powder diffraction pattern (CuKα) of a preferred polymorph of anatabine gluterate.

The schematic drawings are presented for purposes of illustration and not limitation.

### EXAMPLES

### Preparation of anatabine glutarate

Anatabine free base was converted to 1:1 anatabine glutarate by the following methods:
a) To a solution of glutaric acid (16.5 g, 125 mmol, 1.00 eq) in acetonitrile (500 mL) was added anatabine (20.0 g, 125 mmol, 1.00 eq) drop-wise at 25 °C, and the mixture was stirred at 25°C for 1 hour. TLC (Dichloromethane: Methanol = 20:1) showed anatabine (Rf = 0.5) was consumed. The mixture was filtered. The filter cake was collected and concentrated to dryness to give anatabine glutarate (30.0 g, 103 mmol, 82.2% yield, 100% purity) as an off-white solid.
b) To a solution of anatabine (11.6 g, 72 mmol) in acetonitrile (700 ml) was added glutaric acid (9.6 g, 72 mmol). The reaction mixture became cloudy. The reaction mixture was then heated until a clear yellow solution was obtained. The mixture was allowed to cool to room temperature (20°C) and was stirred for 2 hours. A gummy solid appeared which was scratched with a spatula. The mixture was stirred for a further 30 minutes, and the resulting pale-yellow solid was filtered under an atmosphere of argon, washed with acetonitrile (500 ml) and dried under reduced pressure at 45°C for 45 minutes to give anatabine glutarate (18.3 g, 87%) as a pale yellow solid.

¹HNMR (D₂O), δ: 8.84-8.45 (m, 2H), 7.99 (d, J = 7.8 Hz, 1H), 7.59-7.55 (m, 1H), 6.08 (d, J = 8.4 Hz, 1H), 5.85 (d, J = 10.4 Hz, 1H), 4.63-4.59 (m, 1H), 3.97-3.87 (m, 1H), 3.81-3.70 (m, 1H), 2.80-2.53 (m, 2H), 2.25 (t, J = 7.6 Hz, 4H), 1.82-1.74 (m, 2H). The chemical purity of anatabine glutarate was assessed using Waters Acquity UPLC H-class with PDA detector and SQD mass spectrometer, column BEH C18, 2.1 x 50 mm, 1.7 µM running a gradient with detection at 261 nM. The retention time of anatabine glutarate was 1.125 min and purity 99.41%, [M+H]⁺ 161.0. Comparison of FTIR spectra of anatabine glutarate and anatabine free base indicates change of N-H band suggesting confirmation of salt formation.

The anatabine glutarate obtained in this manner was recrystallized from 2.5 mL actonitrile while cooling down after having been heated to reflux. The solid phase was recovered and dried.

The anatabine glutarate salt was analyzed by X-ray powder diffraction (XRPD) between 2-40° 2Θ using zero background silicone wafers (with 9 mm cavities). It was found to have a purity higher than 99% by uHPLC. FIG. 1 is an X-ray powder diffraction pattern (CuKα) of a preferred polymorph of anatabine gluterate.

### Preparation of anatabine powder compositions

Anatabine glutarate is a solid salt. The use of such solid salt during spray drying helps to limit losses, stabilizes the powder obtained, and enables production of powders with high effective content of anatabine glutarate.

Two different batches of spray dried anatabine powder formulation were produced and the results are provided below.

For the manufacture of these spray dried formulations, the following raw materials were used:
1) Anatabine glutarate solid salt; a 2% (effective) anatabine (3.62% of anatabine glutarate) formulation (i.e. for a multiple inhalation use=> i.e. 50 mg of powder to deliver 1 mg of anatabine) and a 10% (effective) anatabine (18.17% anatabine glutarate) formulation (i.e. for single straight inhalation use => i. e. 10 mg of powder in the capsule to deliver 1 mg) were made. Stoichiometric ratio of anatabine and glutaric acid is 1:1.
2) L-Leucine; its use is beneficial for the powder flowability, but it is not compulsory. It is possible to manufacture an anatabine powder formulation with/without leucine (optional). 10% w/w L-Leucine.
3) Trehalose Dihydrate; used as excipient. 86.35% w/w Trehalose or 71.76% w/w Trehalose.

Spray drying feed solutions were prepared in deionised (DI) water and spray dried immediately after preparation. Spray drying was performed using a Buchi B-290 spray dryer fitted with a Buchi two-fluid nozzle, and standard Buchi cyclone. Feed solutions were protected from light during processing. All powders were handled under reduced humidity (<30% RH) and stored in sealed glass amber jars at 2-8 °C.

Spray drying conditions on Buchi B-290 spray dryer. Atomization Pressure 6 bar. Target Outlet Temperature 80°C. Aspirator 100%. Feed rate 3 g/min.

Particle size analysis of spray dried powders was performed using a Sympatec HELOS particle size analyser equipped with an R3 lens (range 0.5 to 175 micrometers) and an ASPIROS dispersion unit. Dispersal was achieved using compressed air at a pressure of 1 bar. Measurements were made in triplicate and mean data reported.

Blending and Micronisation. 6.500g of spray dried formulation was blended with 1.147 g of leucine for five minutes at 36 rpm using a turbula mixer. The resulting blend, 85% w/w spray dried formulation and 15% w/w leucine, was micronised using an Atritor M3 fluid energy mill under the following conditions: Venturi Pressure 8.0 bar. Grinding Pressure 3 - 4 bar. Feed rate 2.4 g/min.

Two spray dryer feed solutions were prepared according to Table 1 below. Trehalose was added as trehalose dihydrate, the loss of the water of hydration during processing was compensated for in all calculations.

**Table 1**

| Example | Total Solids (g) | DI water (ml) | Anatabine Gluterate (g) | L-Leucine (g) | Trehalose (g) |
|---|---|---|---|---|---|
| 1 | 25 | 250 | 0.912 | 2.5 | 23.9 |
| 2 | 25 | 250 | 4.561 | 2.5 | 19.8 |

### Results

| Example | Formulation | X₁₀ (µm) | X₅₀ (µm) | X₉₀ (µm) | VMD (µm) |
|---|---|---|---|---|---|
| 1 | Anatabine Glutarate (3.65%); leucine (10%); Trehalose (86.35%) | 0.99 | 2.89 | 6.04 | 3.31 |
| 2 | Anatabine Glutarate (18.24%); leucine (10%); Trehalose (71.76%) | 0.99 | 2.77 | 5.63 | 3.09 |

Both Example 1 and Example 2 spray dried successfully with processing yields of about 85%. The spray dried materials of Example 1 and Example 2 were both noted as fine, free-flowing white powder.
For the purpose of the present description and of the appended claims, except where otherwise indicated, all numbers expressing amounts, quantities, percentages, and so forth, are to be understood as being modified in all instances by the term "about." Also, all ranges include the maximum and minimum points disclosed and include any intermediate ranges therein, which may or may not be specifically enumerated herein. In this context, therefore, a number "A" is understood as A ±2% of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

## Claims

1. An inhalable powder composition comprising:
a plurality of particles comprising an anatabine compound and a sugar or adhesion reducing compound, the plurality of particles having a particle size of about 20 micrometres or less, or of about 10 micrometres or less, or from about 1 to about 4 micrometres, or of about 20 micrometres to about 200 micrometres measured as mass medium aerodynamic diameter with a cascade impactor.

2. The powder composition according to claim 1, wherein the anatabine compound comprises an anatabine salt.

3. The powder composition according to claim 1, wherein the anatabine compound comprises an anatabine glutarate.

4. The powder composition according to any preceding claim, wherein the plurality of particles comprise an amorphous sugar.

5. The powder composition according to claim 4, wherein the sugar comprises lactose, sucrose, raffinose, trehalose, fructose, dextrose, glucose, maltose, or combinations thereof, preferably sugar comprises trehalose.

6. The powder composition according to any preceding claim, wherein the adhesion reducing compound comprises an amino acid or peptide.

7. The powder composition according to any preceding claim, wherein the adhesion reducing compound comprises histidine, alanine, isoleucine, arginine, leucine, asparagine, lysine, aspartic acid, methionine, cysteine, phenylalanine, glutamic acid, threonine, glutamine, tryptophan, glycine, valine, pyrrolysine, proline, selenocysteine, serine, tyrosine, or a combination thereof, preferably wherein the amino acid comprises leucine.

8. The powder composition according to any preceding claim, wherein the plurality of particles comprise from 50 to 99% sugar by weight, or from 70 to 90% sugar by weight, or from 70 to 80% sugar by weight, or from 80 to 90% sugar by weight, or from 80 to 85% sugar by weight.

9. The powder composition according to any preceding claim, wherein the plurality of particles comprise from 0.5 to 20% anatabine compound by weight, or from 1 to 10% anatabine compound by weight, or from 10 to 20% anatabine compound by weight, or from 1 to 5% anatabine compound by weight, or from 5 to 15% anatabine compound by weight.

10. The powder composition according to any preceding claim, wherein the plurality of particles comprise from 0.5 to 20% amino acid by weight, or from 1 to 15% amino acid by weight, or from 1 to 10% amino acid by weight.

11. The powder composition according to any preceding claim, wherein the plurality of particles comprise 0.5 to 20% anatabine glutarate, from 70 to 90% sugar, and from 0 to 20% leucine, all by weight.

12. The powder composition according to any preceding claim, wherein the plurality of particles comprise 0.5 to 20% anatabine glutarate, from 70 to 90% sugar, and from 1 to 20% leucine, all by weight, preferably the plurality of particles comprise 1 to 10% anatabine glutarate, from 70 to 90% sugar, and from 1 to 10% leucine, all by weight.

13. The powder composition according to any preceding claim, further comprising a second plurality of particles having a particle size of 20 micrometers or greater, or from 50 micrometres to 200 micrometres.

14. A method of forming a powder composition, comprising:
combining an anatabine compound and a sugar, in a liquid carrier, to form a liquid mixture; and
spray drying the liquid mixture to form a plurality of particles, or
spray drying the liquid mixture to form a plurality of particles having a first size, and then micronizing the plurality of particles having a first size to a reduced size.

## Patentansprüche

1. Inhalierbare Pulverzusammensetzung, umfassend:
eine Mehrzahl von Partikeln, umfassend eine Anatabinverbindung und einen Zucker oder eine haftungsmindernde Verbindung, die Mehrzahl von Partikeln eine Partikelgröße von etwa 20 Mikrometer oder weniger oder von etwa 10 Mikrometer oder weniger oder von etwa 1 bis etwa 4 Mikrometer oder von etwa 20 Mikrometer bis etwa 200 Mikrometer, gemessen als aerodynamischer Durchmesser in der Massenmitte mit einem Kaskadenimpaktor, aufweisend.

2. Pulverzusammensetzung nach Anspruch 1, wobei die Anatabinverbindung ein Anatabinsalz umfasst.

3. Pulverzusammensetzung nach Anspruch 1, wobei die Anatabinverbindung ein Anatabinglutarat umfasst.

4. Pulverzusammensetzung nach einem beliebigen vorhergehenden Anspruch, wobei die Mehrzahl von Partikeln einen amorphen Zucker umfasst.

5. Pulverzusammensetzung nach Anspruch 4, wobei der Zucker Lactose, Saccharose, Raffinose, Trehalose, Fructose, Dextrose, Glucose, Maltose oder Kombinationen davon umfasst, der Zucker bevorzugt Trehalose umfassend.

6. Pulverzusammensetzung nach einem beliebigen vorhergehenden Anspruch, wobei die haftungsmindernde Verbindung eine Aminosäure oder ein Peptid umfasst.

7. Pulverzusammensetzung nach einem beliebigen vorhergehenden Anspruch, wobei die haftungsmindernde Verbindung Histidin, Alanin, Isoleucin, Arginin, Leucin, Asparagin, Lysin, Asparaginsäure, Methionin, Cystein, Phenylalanin, Glutaminsäure, Threonin, Glutamin, Tryptophan, Glycin, Valin, Pyrrolysin, Prolin, Selenocystein, Serin, Tyrosin oder eine Kombination davon umfasst, bevorzugt wobei die Aminosäure Leucin umfasst.

8. Pulverzusammensetzung nach einem beliebigen vorhergehenden Anspruch, wobei die Mehrzahl von Partikeln 50 bis 99 Gew.-% Zucker oder 70 bis 90 Gew.-% Zucker oder 70 bis 80 Gew.-% Zucker oder 80 bis 90 Gew.-% Zucker oder 80 bis 85 Gew.-% Zucker umfasst.

9. Pulverzusammensetzung nach einem beliebigen vorhergehenden Anspruch, wobei die Mehrzahl von Partikeln 0,5 bis 20 Gew.-% Anatabinverbindung oder 1 bis 10 Gew.-% Anatabinverbindung oder 10 bis 20 Gew.-% Anatabinverbindung oder 1 bis 5 Gew.-% Anatabinverbindung oder 5 bis 15 Gew.-% Anatabinverbindung umfasst.

10. Pulverzusammensetzung nach einem beliebigen vorhergehenden Anspruch, wobei die Mehrzahl von Partikeln 0,5 bis 20 Gew.-% Aminosäure oder 1 bis 15 Gew.-% Aminosäure oder 1 bis 10 Gew.-% Aminosäure umfasst.

11. Pulverzusammensetzung nach einem beliebigen vorhergehenden Anspruch, wobei die Mehrzahl von Partikeln 0,5 bis 20 Gew.-% Anatabinglutarat, 70 bis 90 Gew.-% Zucker und 0 bis 20 Gew.-% Leucin umfasst.

12. Pulverzusammensetzung nach einem beliebigen vorhergehenden Anspruch, wobei die Mehrzahl von Partikeln 0,5 bis 20 Gew.-% Anatabinglutarat, 70 bis 90 Gew.-% Zucker und 1 bis 20 Gew.-% Leucin umfasst. bevorzugt die Mehrzahl der Partikel 1 bis 10 Gew.-% Anatabinglutarat, 70 bis 90 Gew.-% Zucker und 1 bis 10 Gew.-% Leucin umfasst.

13. Pulverzusammensetzung nach einem beliebigen vorhergehenden Anspruch, ferner umfassend eine zweite Mehrzahl von Partikeln, aufweisend eine Partikelgröße von 20 Mikrometer oder mehr oder von 50 Mikrometer bis 200 Mikrometern.

14. Verfahren für ein Bilden einer Pulverzusammensetzung, umfassend:
Kombinieren einer Anatabinverbindung und eines Zuckers in einem flüssigen Träger, um ein flüssiges Gemisch zu bilden; und
Sprühtrocknen des flüssigen Gemisches, um eine Mehrzahl von Partikeln zu bilden, oder
Sprühtrocknen des flüssigen Gemisches, um eine Mehrzahl von Partikeln zu bilden, die eine erste Größe aufweisen, und anschließendes Mikronisieren der Mehrzahl von Partikeln, die eine erste Größe aufweisen, auf eine reduzierte Größe.

## Revendications

1. Composition de poudre inhalable comprenant :
une pluralité de particules comprenant un composé d'anatabine et un sucre ou composé réducteur d'adhérence, la pluralité de particules ayant une granulométrie d'environ 20 micromètres ou moins, ou d'environ 10 micromètres ou moins, ou d'environ 1 à environ 4 micromètres, ou d'environ 20 micromètres à environ 200 micromètres mesurée en tant que diamètre aérodynamique moyen en masse avec un impacteur en cascade.

2. Composition de poudre selon la revendication 1, dans laquelle le composé d'anatabine comprend un sel d'anatabine.

3. Composition de poudre selon la revendication 1, dans laquelle le composé d'anatabine comprend un sel de glutarate.

4. Composition en poudre selon l'une quelconque des revendications précédentes, dans laquelle la pluralité de particules comprend un sucre amorphe.

5. Composition de poudre selon la revendication 4, dans laquelle le sucre comprend du lactose, du saccharose, du raffinose, du tréhalose, du fructose, du dextrose, du glucose, du maltose ou des combinaisons de ceux-ci, de préférence le sucre comprend du tréhalose.

6. Composition de poudre selon l'une quelconque des revendications précédentes, dans laquelle le composé réducteur d'adhérence comprend un acide aminé ou un peptide.

7. Composition en poudre selon l'une quelconque des revendications précédentes, dans laquelle le composé réducteur d'adhérence comprend l'histidine, l'alanine, l'isoleucine, l'arginine, la leucine, l'asparagine, la lysine, l'acide aspartique, la méthionine, la cystéine, la phénylalanine, l'acide glutamique, la thréonine, la glutamine, le tryptophane, la glycine, la valine, la pyrrolysine, la proline, la sélénocystéine, la sérine, la tyrosine, ou une combinaison de ceux-ci, de préférence dans laquelle l'acide aminé comprend la leucine.

8. Composition de poudre selon l'une quelconque des revendications précédentes, dans laquelle la pluralité de particules comprend de 50 à 99 % en poids de sucre, ou de 70 à 90 % en poids de sucre, ou de 70 à 80 % en poids de sucre, ou de 80 à 90 % en poids de sucre, ou de 80 à 85 % en poids de sucre.

9. Composition de poudre selon l'une quelconque des revendications précédentes, dans laquelle la pluralité de particules comprend de 0,5 à 20 % en poids de composé d'anatabine, ou de 1 à 10 % en poids de composé d'anatabine, ou de 10 à 20 % en poids de composé d'anatabine, ou de 1 à 5 % en poids de composé d'anatabine, ou de 5 à 15 % en poids de composé d'anatabine.

10. Composition de poudre selon l'une quelconque des revendications précédentes, dans laquelle la pluralité de particules comprend de 0,5 à 20 % en poids d'acide aminé, ou de 1 à 15 % en poids d'acide aminé, ou de 1 à 10 % en poids d'acide aminé.

11. Composition de poudre selon l'une quelconque des revendications précédentes, dans laquelle la pluralité de particules comprend 0,5 à 20 % de glutarate d'anatabine, 70 à 90 % de sucre et 0 à 20 % de leucine, tous en poids.

12. Composition de poudre selon l'une quelconque des revendications précédentes, dans laquelle la pluralité de particules comprend 0,5 à 20 % de glutarate d'anatabine, 70 à 90 % de sucre et 1 à 20 % de leucine, tous en poids, de préférence la pluralité de particules comprend 1 à 10 % de glutarate d'anatabine, 70 à 90 % de sucre et 1 à 10 % de leucine, tous en poids.

13. Composition de poudre selon l'une quelconque des revendications précédentes, comprenant en outre une deuxième pluralité de particules ayant une granulométrie de 20 micromètres ou plus, ou de 50 micromètres à 200 micromètres.

14. Procédé de formation d'une composition de poudre, comprenant :
la combinaison d'un composé d'anatabine et d'un sucre, dans un véhicule liquide, pour former un mélange liquide ; et
le séchage par pulvérisation du mélange liquide pour former une pluralité de particules, ou
le séchage par pulvérisation du mélange liquide pour former une pluralité de particules ayant une première taille, puis la micronisation de la pluralité de particules ayant une première taille à une taille réduite.
